# EUROPEAN PATENT SPECIFICATION

(11) **EP 3 875 109 B1**
(45) Date of publication and mention of the grant of the patent: **02.10.2024**
(21) Application number: 19878870.5
(22) Date of filing: 29.03.2019
(51) Int. Cl.: A61K 38/48, A61P 7/06

(54) **ANTI-PLATELET THROMBOLYSIN FOR USE IN THE TREATMENT OF SICKLE CELL ANEMIA**
ANTITHROMBOZYTENTHROMBOLYSIN ZU VERWENDUNG IN DER BEHANDLUNG VON SICHELZELLENANÄMIE
THROMBOLYSINE ANTIPLAQUETTAIRE POUR L'UTILISATION DANS LE TRAITEMENT DE LA DRÉPANOCYTOSE

(30) Priority: 29.10.2018 CN 201811267000
(43) Date of publication of application: 08.09.2021
(73) Proprietor: Zhaoke Pharmaceutical (Hefei) Company Limited, Anhui 230088 (CN)
(72) Inventor: CHO, Jaehyung, Hefei, Anhui 230088 (CN); LI, Xiaoyi, Hefei, Anhui 230088 (CN); DAI, Xiangrong, Hefei, Anhui 230088 (CN)
(74) Representative: Isern Patentes y Marcas S.L.
(86) International application number: PCT/CN2019/080448
(87) International publication number: WO 2020/087856

(56) References cited:
- CN-A- 101 838 323
- CN-A- 105 833 255
- CN-A- 109 464 659
- LI JING ET AL: "Platelet Protein Disulfide Isomerase Promotes Glycoprotein Ib[alpha]-Mediated Platelet-Neutrophil Interactions Under Thromboinflammatory Conditions", CIRCULATION, vol. 139, no. 10, 5 March 2019 (2019-03-05), US, pages 1300 - 1319, XP055926299, ISSN: 0009-7322, DOI: 10.1161/CIRCULATIONAHA.118.036323
- "Posters (Abstracts 264-2239)", HEPATOLOGY, JOHN WILEY & SONS, INC, US, vol. 66, 1 October 2017 (2017-10-01), pages 149 - 1185, XP071563194, ISSN: 0270-9139, DOI: 10.1002/HEP.29501

## Description

### FIELD

The invention relates to an anti-platelet thrombolysin for use in the treatment of sickle cell anemia.

### BACKGROUND

Anemia is a common clinical symptom, which refers to a state in which the total amount of red blood cells in the human blood decreases below the normal value. It can be caused by a variety of system diseases. Because the measurement of red blood cell volume is more complicated, hemoglobin Hb concentration is often used clinically instead.

Based on different clinical characteristics, there are different classifications of anemia. According to the progression speed of anemia, it is divided into acute and chronic anemia; according to red blood cell morphology, it is divided into macrocytic anemia, normal cell anemia and small cell hypochromic anemia; according to hemoglobin concentration, it is divided into mild, moderate, severe and very severe anemia; according to bone marrow red line of proliferation, it is divided into proliferative anemia, including hemolytic anemia, iron deficiency anemia, megaloblastic anemia, and hyperplastic hypoplastic anemia such as aplastic anemia.

Clinically, according to its pathogenesis and etiology, there are mainly:

Anemia with reduced erythropoiesis due to abnormal hematopoietic cells, bone marrow hematopoietic microenvironment and hematopoietic raw materials affect red blood cell production, such as aplastic anemia, pure red blood cell aplastic anemia, congenital abnormal red blood cell production anemia, hematopoietic malignant clonal anemia, etc.
2) Anemia caused by abnormal hematopoietic microenvironment, including anemia caused by bone marrow stromal and stromal cell damage, anemia caused by abnormal levels of hematopoietic regulatory factors, and autoimmune hemolytic anemia caused by lymphocyte hyperfunction, such as sickle cell anemia and anemia caused by hyperapoptosis;
3) Anemia caused by insufficient hematopoietic materials or utilization disorders, including anemia caused by folic acid or vitamin B12 deficiency or utilization disorders, anemia caused by iron deficiency and iron utilization disorders;
4) Anemia of excessive red blood cell destruction, including anemia caused by abnormal red blood cells and abnormal surrounding environment of red blood cells;
5) Hemorrhagic anemia, etc.

The cause of anemia, the decrease in capacity of blood for carrying oxygen, the decrease in blood volume, the speed at which anemia occurs, and the compensatory and tolerance capabilities of the blood, circulation, and respiratory systems all affect the clinical manifestations of anemia. The severity of anemia symptoms depends on the speed of anemia, the degree of anemia and the body's ability to compensate.

Sickle cell anemia (also known as sicklemia, Sickle Cell Disease, SCD) is a potentially fatal human genetic disease, autosomal inherited hemoglobinopathy, with clinical manifestations of chronic hemolytic anemia, susceptibility to infection and recurring pain, resulting in chronic ischemia causing organ and tissue damage. The sickle red blood cells are stiff and have poor deformability. They can be hemolyzed by the destruction of vascular mechanisms and phagocytosis by the mononuclear macrophage system, and can easily block capillaries and cause local hypoxia and inflammation.

Sickle cell anemia is also a disease that seriously endangers the health of mothers and children, causing the fetal mortality rate to reach 5% and the maternal mortality rate to 4.62%. Because the amino acid glutamic acid at position 6 of the β-peptide chain is replaced by valine, it forms sickle hemoglobin (HbS), which replaces normal Hb (HbA). Normal adult hemoglobin is a tetramer composed of two alpha chains and two beta chains. The alpha chain and the beta chain are composed of 141 and 146 amino acid, respectively. In patients with sickle cell anemia, glutamic acid at the 6th position of the β chain is replaced by valine, forming abnormal hemoglobin S (HbS), which replaces normal hemoglobin (HbA), and when the partial pressure of oxygen drops intermolecular interactions of HbS form a spiral-shaped polymer with very low solubility, which makes red blood cells twisted into sickle cells, i.e., sickling. Many factors such as the concentration of HbS in red blood cells, the degree of deoxygenation, acidosis, and the degree of red blood cell dehydration are related to red blood cell sickling. The initial stage of sickling of red blood cells is reversible, and oxygen can be used to reverse the sickling process. But when the sickle has severely damaged the red blood cell membrane, the sickle becomes irreversible. Even if the cells are placed under aerobic conditions, the red blood cells remain sickle-shaped. Sickled red blood cells can also increase blood viscosity, slow blood flow. Due to its poor deformability, it is easy to block capillaries, causing local hypoxia and inflammation, leading to painful crises in the corresponding parts, which mostly occur in muscles, bones, limbs and joints, chest and abdomen, especially joints and chest and abdomen are common.

When blood is deoxygenated in patients with sickle cell anemia, the cells lose water and deform. In addition, the adhesion of red blood cells to capillary endothelial cells increases, especially the vascular endothelial cells that fall into the blood promote the adhesion process, sickle cells greatly increased, these cells are extremely fragile and easily damaged, resulting in low levels of hemoglobin. The serious consequence is that the capillaries of certain organs are blocked by abnormal cells, which aggravates blood vessel obstruction and hemolysis. This is the main cause of death for many sickle cell anemia patients. CN105833255 discloses treatment of thromobotic thrombocytopenic purpura (TTP) with antiplatelet thrombolysin, addressing in the treatment of schistocyte hemolytic anemia. However, sickle cell anemia is not characterized by the presence of schistocytes, but by still and abnormally found red blood cells.

In severe cases, hydroxyurea can reduce the number of painful crises and blood transfusions needed, and can also reduce the frequency of chest syndrome. This disease is mainly caused by hypoxia that makes red blood cells sickle and obstructs capillaries, causing a painful crisis. Alternative treatment and vasodilators should be used. The supplementation of folic acid can reduce the increased cysteine level and improve the function of vascular endothelium. There are also bone marrow transplantation and fetal liver hematopoietic stem cell transplantation to save patients and improve the quality of life. The indirect flow blood cell separator is used to separate the patient's red blood cells and at the same time the blood donor's glycerol-free red blood cells are infused, and a better effect can be obtained. There are also advocates for partial exchange of blood while intravenous infusion of 5% glucose to reduce blood viscosity. Low-dose bicoumarin treatment can reduce the occurrence of prethrombotic conditions in patients. There is no clear genetic change in the genetic makeup of the affected individual, so etiological treatment of the disease is meaningless. The current conventional treatment is to prevent hypoxia, dehydration, and infection to relieve symptoms, reduce organ damage complications, promote hematopoiesis and prolong life. CN105833255 discloses the treatment of thrombotic thrombocytopenic purpura with antiplatelet thrombolysin made up of an α chain, and a β chain.

### SUMMARY

The inventor of the present patent unexpectedly discovered that antiplatelet thrombolysin can significantly reduce platelet-neutrophil aggregation in anemic mice, prolong the survival time of anemic mice, and is expected to be applied to the treatment of sickle cell anemia.

In view of this, the purpose of the present invention is to provide a new use of antiplatelet thrombolysin. The antiplatelet thrombolysin (APT, Anfibatide) of the present invention is a C-type lectin-like protein isolated from the snake venom of Agkistrodon acutus. It is composed of two peptide chains of α chain and β chain. The α chain amino acid sequence of the amino acid sequence is shown in SEQ ID NO. 1, and the β chain amino acid sequence is shown in SEQ ID NO. 2; and the antiplatelet thrombolysin has an activity of treating sickle cell anemia.

The object of the invention is antiplatelet thrombolysin for use in treatment of sickle cell anemia, wherein said antiplatelet thrombolysin consists of two peptide chains of an α chain and a β chain, and the α chain amino acid sequence is shown in SEQ ID NO.1, the β chain amino acid sequence is shown in SEQ ID NO. 2.

Preferably, the antiplatelet thrombolysin for use according to the present invention is present in a medicament together with a pharmaceutically acceptable excipient.

Preferably, the antithrombolysin for use according to the present invention is provided as an external preparation or an injection.

More preferably, the external preparation is tincture, ointment, cream, lotion, rinse, liniment or gel.

The use of the antiplatelet thrombolysin provided in the present invention for treating sickle cell anemia is not the same as the use published in the prior art, and can also supplement the deficiencies of existing drug treatments. *In vivo* and *in vitro* experiments have confirmed that antiplatelet thrombolysin can significantly reduce platelet-neutrophil aggregation in a TNF-α-induced sickle cell anemia model, and prolong the survival time of living organisms with TNF-α-induced sickle cell anemia.

### BRIEF DESCRIPTION OF DRAWINGS

Figure 1 shows the interaction between neutrophils in a mouse model of sickle cell anemia;
Figure 2 shows the interaction between platelets and neutrophils in a mouse model of sickle cell anemia;
Figure 3 is a representative image of intravital microscopy, from left to right are BSA group, IgG group, antiplatelet thrombolysin group, antiPDI group, antiPDI+antiplatelet thrombolysin group, and the big arrow and the small arrow show blood flow and the rolling direction of neutrophils, respectively;
Figure 4 shows the survival time of sickle cell anemia mice after administration.

### DETAILED DESCRIPTION

The present invention provides antiplatelet thrombolysin for use in treatment of sickle cell anemia, wherein said antiplatelet thrombolysin consists of two peptide chains of an α chain and a β chain, and the α chain amino acid sequence is shown in SEQ ID NO. 1, the β chain amino acid sequence is shown in SEQ ID NO. 2. Those skilled in the art can achieve it in view of this disclosure and appropriate improvement of the process parameters. The use of the present invention has been described through the preferred examples.

The reagents used in the present invention are all common commercially available products, and all are available in the market.

The following examples further illustrate the present invention:

### Example 1 Anti-platelet thrombolysin reduces platelet-neutrophil aggregation in a mouse model of sickle cell anemia induced by TNF-α

Neutrophils (neutrophils) play a very important role in the non-specific cellular immune system of the blood. Activated neutrophils reach the diseased site through chemotaxis, and the bactericidal substances they carry will be released locally. Through their respective destructive effects, they will cause tissue damage and further lead to organ disorders. After neutrophils invade an organ, they will induce the accumulation of neutrophils in other important organs, resulting in multiple organ damage. At the same time, platelet selective receptors mediate the binding and adhesion of activated platelets and neutrophils, neutrophils aggregate, and accelerate tissue damage; on the other hand, platelets adhere to neutrophils, promote platelet aggregation, and mediate vascular occlusion. At the same time, the cell membrane of neutrophils can release tetraenoic acid. Under the action of enzymes, thromboxane and prostaglandins produced by tetraenoic acid have a significant effect on regulating the diameter and permeability of blood vessels, and can also cause inflammation and pain. The sickle red blood cells in the blood of sickle cell anemia are stiff and have poor deformability, and they are easy to block capillaries and cause local hypoxia and inflammation. The clinical manifestations are chronic hemolytic anemia, easy interference and recurrent pain, and ischemia leads to organ and tissue damage. The current routine treatment of sickle cell anemia is to prevent hypoxia, dehydration, and infection to relieve symptoms, reduce organ damage complications, promote hematopoiesis and prolong life.

By transplanting the bone marrow of Berkeley mice into 6-week-old WT mice that have been irradiated with a lethal dose, chimeric control and Berkeley mice were generated. Three months after transplantation, the chimera was confirmed by PCR and electrophoresis analysis. These chimeric Berkeley Mice are sickle cell anemia mice.

Sickle cell anemia model mouse platelets (2x107 cells/ml) and neutrophils (1x106 cells/ml) were taken, and labeled with DyLight488-anti-CD42C and Alexa Fluor647-anti-Ly-6G antibodies. Human neutrophils and platelets were respectively labeled with Alexa Fluor 488-anti-CD41 and FITC-anti-L-selectin antibodies. Neutrophils were induced with 20ng/ml TNF-α for 5 minutes, and platelets were pre-incubated with control IgG (10µg/ml) and anti PDI antibodies (protein disulfide bond isomerase inhibitor, 10µg/ml), BSA (0.2µg/ml), antiplatelet thrombolysin (0.2µg/ml) and antiplatelet thrombolysin + antiPDI for 30min respectively at room temperature, and then incubated with 0.025U/ml thrombin at 37°C for 5 minutes, and then incubated with 50uM PPACK, stirred and mixed with platelets and neutrophils at 1000 rpm, fixed after 5 minutes and analyzed by flow cytometry.

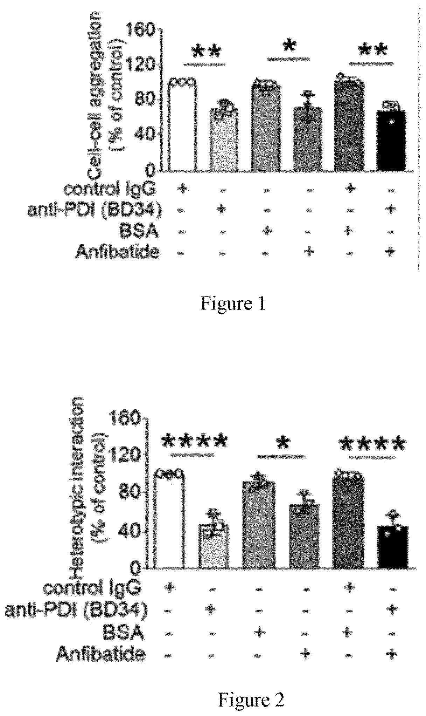

During pathology, platelets adhere to neutrophils, promote platelet aggregation, mediate vascular occlusion, and aggravate pathophysiological reactions. The test results showed that 0.2µg/ml of antiplatelet thrombolysin can reduce the aggregation of neutrophils in sickle cell anemia model mice (Figure 1), and it can also reduce the combination of platelets and neutrophils (Figure 2), thereby playing a role in inhibiting blood vessel damage.

In Figure 1, the ordinate is the cell-cell interaction relative to the control group, and the abscissa is the experimental group from left to right: control IgG group, anti PDI group, BSA group, anfibatide group, IgG+BSA group, anti PDI+ anfibatide group.

In Figure 2, the ordinate is the interaction between platelets and neutrophils relative to the control group, and the abscissa is the experimental group from left to right: control IgG group, anti PDI group, BSA group, anfibatide group, IgG+BSA group, anti PDI+anfibatide group.

The experimental data was statistically analyzed by ANOVA and Tukey's test.
n=4, *P <0.05, **P <0.01, or ***P <0.001

### Example 2 Therapeutic effect of antiplatelet thrombolysin on mice with sickle cell anemia

By transplanting the bone marrow of Berkeley mice into 6-week-old WT mice that have been irradiated with a lethal dose, chimeric control and Berkeley mice were generated. Three months after transplantation, the chimera was confirmed by PCR and electrophoresis analysis. These chimeric Berkeley Mice are sickle cell anemia mice. Successfully modeled mice were injected with 500ng TNF-α through intraperitoneal injection to induce severe inflammation. After 3 hours of induction, single-blind administration was used. The administration groups included: IgG control (1.5µg/g), antiPDI (BD34, 1.5µg/g), BSA (25ng/g), anfibatide (25ng/g), antiPDI (1.5µg/g) + anfibatide (25ng/g).

After administration, the mice were observed by intravital microscope. During the study, the survival time of each mouse was recorded. The recording time started from the injection of TNFα and ended when the mice died or 6h after injection of TNF-α. GraphPad Prism 7 software was used to analyze the data. Statistical analysis was performed by Student's t-test and ANOVA. n = 8/group, * P <0.05, ** P <0.01, *** P <0.001, **** P <0.0001.

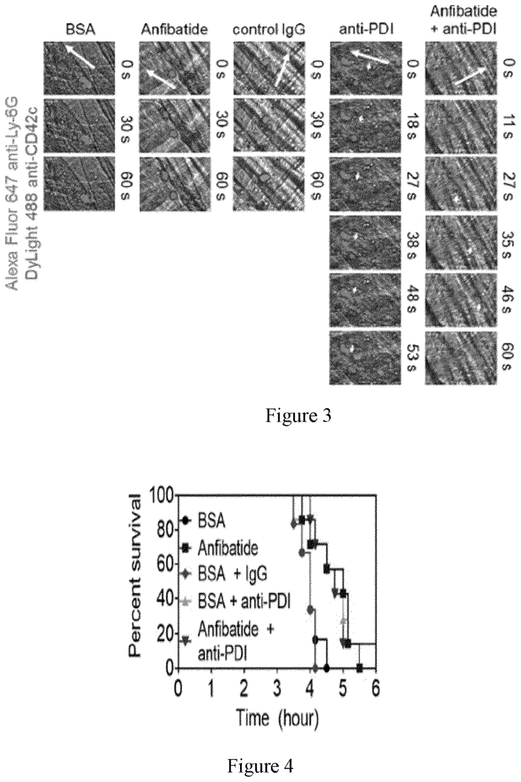

The results showed that, compared with the BSA control group, 25ng/g antiplatelet thrombolysin eliminated the adhesion of platelets and neutrophils on the blood vessel wall of SCD mice by intravital microscope observation (Figure 3). DyLight488-anti-CD42C and Alexa Fluor647-anti-Ly-6G antibody were used in the figure to label the platelets and neutrophils respectively. The large arrow indicates the direction of blood flow, and the small arrow indicates the rolling direction of neutrophils. The TNF-α stimulation and intravital microscopy surgery can cause acute vascular embolism, leading to the death of most SCD mice. The experimental observation period is the death of mice or 6 hours after the administration of TNFα.

Compared with the control groups, P = 0.0249 for the comparison between BSA and anti-platelet thrombolysin group, P = 0.028 for the comparison between BSA + IgG and BSA + anti-PDI group, P = 0.0165 for the comparison between BSA + IgG and anti-platelet thrombolysin anti-PDI group. 50% survival time of mice treated with antiplatelet thrombolysin (Anfibatide) was 5.0h, which was the longest survival time of mice in all administration groups (see Figure 4), showing that antiplatelet thrombolysin has effective improving effect against sickle cell anemia in living bodies.

## Claims

1. Antiplatelet thrombolysin for use in treatment of sickle cell anemia, wherein said antiplatelet thrombolysin consists of two peptide chains of an α chain and a β chain, and the α chain amino acid sequence is shown in SEQ ID NO.1, the β chain amino acid sequence is shown in SEQ ID NO. 2.

## Patentansprüche

1. Antithrombozytenthrombolysin zur Verwendung in der Behandlung von Sichelzellenanämie, wobei das Antithrombozytenthrombolysin aus zwei Peptidketten einer α-Kette und einer β-Kette besteht und die Aminosäuresequenz der α-Kette in SEQ ID NO. 1 gezeigt ist, die Aminosäuresequenz der β-Kette in SEQ ID NO. 2 gezeigt ist.

## Revendications

1. Thrombolysine antiplaquettaire destinée à être utilisée dans le traitement de l'anémie falciforme, dans laquelle ladite thrombolysine antiplaquettaire est constituée de deux chaînes peptidiques d'une chaîne α et d'une chaîne β, et la séquence d'acides aminés de la chaîne α est présentée dans la SEQ ID NO.1, la séquence d'acides aminés de la chaîne β est présentée dans la SEQ ID NO. 2.
